# EUROPÄISCHE PATENTSCHRIFT

(11) **EP 1 648 352 B1**
(45) Veröffentlichungstag und Bekanntmachung des Hinweises auf die Patenterteilung: **14.05.2008**
(21) Anmeldenummer: 04707909.0
(22) Anmeldetag: 04.02.2004
(51) Int. Cl.: A61F 2/44, A61F 2/46

(54) **ARNORDNUNG AUS EINER ZERVIKALPROTHESE UND EINSETZINSTRUMENT**
ARRANGEMENT COMPRISING A CERVICAL PROSTHESIS AND AN INSERTION INSTRUMENT
ENSEMBLE COMPRENANT UNE PROTHESE CERVICALE ET UN INSTRUMENT D'INSERTION

(30) Priorität: 15.07.2003 US 619179
(43) Veröffentlichungstag der Anmeldung: 26.04.2006
(73) Patentinhaber: Cervitech, Inc., Rockaway, NJ 07866 (US)
(72) Erfinder: KELLER, Arnold, 23863 Kayhude (DE)
(74) Vertreter: Riesenberg, Axel
(86) Internationale Anmeldenummer: PCT/EP2004/001027
(87) Internationale Veröffentlichungsnummer: WO 2005/007042

(56) Entgegenhaltungen:
- WO-A-01/01893
- WO-A-01/19295
- US-A- 5 314 477

## Beschreibung

Die Erfindung betrifft eine Anordnung aus einer mehrteiligen Zwischenwirbelendoprothese, die eine obere und untere Abschlußplatte und einen Gleitkern dazwischen aufweist, wobei jeder Abschlußplatte ein Paar Aufnahmeöffnungen oder -vorsprünge zugeordnet ist, und einem Einsetzinstrument, das einen Griffbereich und einen Faßbereich mit Haltevorsprüngen oder -öffnungen aufweist, die zum Halten der Zwischenwirbelendoprothese an dem Einsetzinstrument in die Aufnahmeöffnungen oder -vorsprünge einbringbar sind.

Das Dokument WO-A-0119295 offenbart den Oberbegriff des Anspruchs 1.

Zum Implantieren von Zwischenwirbelprothesen sind aufgrund des schwierigen Zugangs zum Implantationsort spezielle Einsetzinstrumente erforderlich. Damit die Zwischenwirbelprothese von dem Einsetzinstrument gehalten werden kann, ist es bekannt, an der ventralen Stirnfläche der Abschlußplatten in Einsetzrichtung weisende Bohrungen vorzusehen, in die passend geformte Stifte des Einsetzinstruments eingreifen (US-A-5,314,477 und WO-A-0119295). Diese Art der Halterung ist zwar recht sicher, jedoch steht der Platzbedarf der Bohrungen einer Verkleinerung der Stirnfläche und damit der gesamten Abschlußplatte im Wege. Für eine Anwendung unter beengten Verhältnissen eignet sich diese Ausführung nur wenig.

Bei einem anderen bekannten (EP-A-1 306 064) Zwischenwirbelprothesen-System sind keine Bohrungen vorgesehen, statt dessen weist das Einsetzinstrument zwei Paare von starr miteinander verbundenen Greifgliedern auf, die jeweils eine Abschlußplatte durch Reibkraft zwischen sich halten. Für sehr kleine Implantate, wie sie im Bereich der Halswirbelsäule verwendet werden und die sehr genau plaziert werden müssen, kann dies zu unsicher sein.

Der Erfindung liegt die Aufgabe zugrunde, eine verbesserte Anordnung aus Zwischenwirbelendoprothese und Einsetzinstrument zu schaffen, die insbesondere an die Bedürfnisse bei der Implantation unter beengten Verhältnisse angepaßt ist, wie sie vor allem im Bereich der Halswirbelsäule auftreten.

Die erfindungsgemäße Lösung liegt in einer Anordnung mit den Merkmalen des Anspruchs 1. Vorteilhafte Weiterbildungen sind Gegenstand der Unteransprüche.

Erfindungsgemäß ist bei einer Anordnung aus einer mehrteiligen Zwischenwirbelendoprothese, die eine obere und untere Abschlußplatte und einen Gleitkern dazwischen aufweist, wobei jeder Abschlußplatte ein Paar Aufnahmeöffnungen oder -vorsprünge zugeordnet ist, und einem Einsetzinstrument, das einen Griffbereich und einen Faßbereich mit Haltevorsprüngen oder -öffnungen aufweist, die zum Halten der Zwischenwirbelendoprothese an dem Einsetzinstrument in die Aufnahmeöffnungen oder -vorsprünge einbringbar sind, vorgesehen, daß die Aufnahmeöffnungen in lateralen Seitenflächen der Zwischenwirbelendoprothese angeordnet sind und zumindest das einer der Abschlußplatten zugeordnete Paar von Aufnahmeöffnungen eine in Richtung zur anderen Abschlußplatte ausgedehnte Form aufweist.

Unter in Richtung zur anderen Abschlußplatte ausgedehnte Form wird verstanden, daß die Aufnahmeöffnung für einen in sie greifenden Haltevorsprung in einer Richtung, nämlich zu der anderen Abschlußplatte, Freiraum läßt, so daß der Haltevorsprung in dieser Richtung gesehen verschieden Positionen einnehmen kann. Man könnte auch sagen, daß die Abschlußplatte wie ein Langloch geformt ist, wobei die Längsachse des Langlochs zur anderen Abschlußplatte weist. Eine zweckmäßige Ausführungsform für eine derart geformte Aufnahmeöffnung ist ein Schlitz.

Mit dieser Ausführung der Aufnahmeöffnungen erlaubt es die erfindungsgemäße Anordnung, Zwischenwirbelendoprotesen mit unterschiedlich dicken Gleitkernen zu fassen und einzusetzen, ohne daß dazu Einstellungen oder Änderungen vorzunehmen wären. Der Chirurg kann so, wenn sich im Laufe einer Operation herausstellt, daß die vorgesehene Zwischenwirbelendoprothese nicht paßt, aus einem Instrumentarium eine andere mit anderer Höhe aussuchen und einfach ohne weiteres mit dem Einsetzinstrument aufnehmen und einsetzen. Das ist insbesondere bei kleinen Zwischenwirbelendoprothesen, z. B. für die Halswirbelsäule, die aufgrund ihrer Kleinheit nur geringe Toleranzen bei der Implantation zulassen, ein erheblicher Vorteil.

Die Anordnung der Aufnahmeöffnungen (oder -vorsprünge) an den lateralen Seitenflächen anstatt wie im Stand der Technik an den Stirnseite der Abschlußplatten verringert den Platzbedarf. Sie erlaubt eine platzsparendere und kleinere Ausführung der Zwischenwirbelendoprothese. Die Anordnung an den lateralen Seitenflächen hat weiter den Vorteil, daß die Haltevorsprünge quer zur Einsetzrichtung angeordnet sind und so die Kraftübertragung beim Einsetzen der Zwischenwirbelendoprothese formschlüssig erfolgt. Die Zwischenwirbelendoprothese ist dank dieser Gestaltung einerseits sicher am Einsetzinstrument gehalten, kann aber andererseits durch Lösen des Formschlusses leicht von dem Einsetzinstrument getrennt werden. Im Stand der Technik ist das anders, dort erfolgt die Kraftübertragung aufgrund der in Einsetzrichtung weisenden Haltevorsprüngen durch Reibschluß. Das bedeutet einen unsichere Positionierung Halt und unerwünscht hohe Lösekräfte, um den Reibschluß zu überwinden. Die Erfindung ermöglicht so eine leichtere Handhabung und eine genauere Positionierung, worauf es besonders bei beengten Platzverhältnissen ankommt.

Vorzugsweise erstreckt sich die eine ausgedehnte Form aufweisende Aufnahmeöffnung über die ganze Höhe der zugeordneten Abschlußplatte. Das ermöglicht einen Ausgleich unterschiedlicher Höhen über einen größeren Bereich. Die eine ausgedehnte Form aufweisende Aufnahmeöffnung kann aber auch an dem Gleitkern oder an beidem angeordnet sein. In letzterem Fall ist es wichtig, daß sie miteinander fluchten. Sie kann sich über einen Teil der Höhe oder vorzugsweise über die gesamte Höhe des Gleitkerns erstrecken.

Es kann zweckmäßig sein, die Form der Aufnahmeöffnung so zu wählen, daß sie sich mit zunehmender Tiefe verjüngt. Dadurch wird das Einführen des entsprechenden Haltevorsprungs erleichtert und es wird trotzdem ein sicherer Sitz erreicht, so daß nur wenig oder gar kein Spiel besteht.

Vorzugsweise weist das der anderen Abschlußplatte zugeordnete Paar von Aufnahmeöffnungen eine konzentrierte Form auf. Bei einer konzentrierten Form steht dem eingreifenden Haltevorsprung anders als bei der ausgedehnten Form kein Freiraum zur Verfügung, d. h. der Haltevorsprung kann nur eine Position einnehmen. Zweckmäßigerweise ist diese Aufnahmeöffnung als eine Rundbohrung ausgebildet. Dadurch wird erreicht, daß die Zwischenwirbelendoprothese eine definierte Position in Bezug auf den darein eingreifenden Haltevorsprung und damit auch in Bezug auf das Einsetzinstrument einnimmt. Unterschiede in den Abmessungen der Zwischenwirbelendoprothese, sei es durch verschiedene Höhen des Gleitkerns oder aufgrund von Herstellungstoleranzen, können dank der erfindungsgemäßen Gestaltung von der Aufnahmeöffnung mit der ausgedehnten Form aufgenommen werden. Die Zwischenwirbelendoprothese kann somit trotz möglicher Unterschiede definiert gehalten werden. Eine unterschiedliche Gestaltung der beiden Paare von Aufnahmeöffnungen hat ferner den Vorteil, daß sie Verwechselungssicherheit gibt. Die Zwischenwirbelendoprothese kann dann nämlich nur mit der passenden Orientierung an dem Einsetzinstrument gehalten sein; eine verkehrte Anordnung wird dadurch vermieden.

Im Regelfall sind die Aufnahmeöffnungen an der Zwischenwirbelendoprothese und die Haltevorsprünge an dem Einsetzinstrument angeordnet. Das ist eine bewährte Anordnung. Die Erfindung ist aber nicht darauf beschränkt, sondern es kann auch vorgesehen sein, daß die Vorsprünge an der Zwischenwirbelendoprothese und die Öffnungen an dem Einsetzinstrument angeordnet sind.

Besonders vorteilhaft ist es, für die Zwischenwirbelendoprothese verschiedene Größen mit unterschiedlich dickem Gleitkern vorzusehen. Damit kann je nach anatomischen Anforderungen die passende Zwischenwirbelendoprothese ausgewählt und mit dem Einsetzinstrument implantiert werden, ohne daß Veränderungen an dem Einsetzinstrument vorgenommen werden müßten. Erkennt der Chirurg im Laufe einer Operation, daß die ursprünglich vorgesehene Zwischenwirbelendoprothese zu hoch oder zu niedrig ist, so braucht er nur eine andere Zwischenwirbelendoprothese passender Höhe auszuwählen und kann sie ohne weiteres einsetzen.

Das Einsetzinstrument ist mit Vorteil als Zange ausgeführt. Die Haltevorsprünge können an den Innenseiten der Backenteile angeordnet sein. Dadurch ist es ermöglicht, durch einfaches Schließen der Zange die Haltevorsprünge in die Aufnahmeöffnungen einzubringen und so die Zwischenwirbelendoprothese an der Zange zu halten. Ferner ermöglicht die Ausbildung als Zange eine raumsparende Konstruktion. Dadurch ergeben sich beträchtliche Vorteile hinsichtlich der Handhabung insbesondere unter beengten Platzverhältnissen wie im Bereich der Halswirbelsäule. Außerdem hat die zangenartige Ausführung den Vorteil, daß mit ihr unterschiedliche Breitenabmessungen der Zwischenwirbelendoprothese einfach ausgeglichen werden können. Dies geschieht, indem die Zange je nach den Breitenabmessungen der Zwischenwirbelendoprothese mehr oder weniger weit geschlossen wird. In Kombination mit der erfindungsgemäßen Gestaltung der Aufnahmeöffnungen können somit sowohl unterschiedliche Breitenabmessungen wie auch Dicken der Zwischenwirbelendoprothese ausgeglichen werden. Das ergibt eine hohe Universalität der Anordnung.

Die Haltevorsprünge sind vorzugsweise plättchenartig und stiftartig ausgeführt. Derart gestaltete Haltevorsprünge erlauben eine gute Anpassung an die Form der Aufnahmeöffnungen und gewährleisten so einen sicheren und spielarmen Sitz der Zwischenwirbelendoprothese an dem Einsetzinstrument.

Es ist zweckmäßig, an einem Faßbereich einen Block mit einer Anschlagfläche zur Anlage an die Zwischenwirbelendoprothese vorzusehen. Beim Aufnehmen der Zwischenwirbelendoprothese mit dem Einsetzinstrument greifen die Haltevorsprünge in die Aufnahmeöffnungen (oder umgekehrt) und bewirken so, daß die Zwischenwirbelendoprothese eine definierte Position zu dem Einsetzinstrument einnimmt. Der Block ist so angeordnet, daß seine Anschlagfläche an der Zwischenwirbelendoprothese anliegt. Die zum Einsetzen der Zwischenwirbelendoprothese erforderlichen Kräfte können dann über die Anschlagfläche auf die Zwischenwirbelendoprothese aufgebracht werden, die Haltevorsprünge brauchen diese in Einsetzrichtung wirkenden Kräfte nicht aufzunehmen. Sie können schwächer dimensioniert und so fein ausgeführt werden, wie es für eine genaue Positionierung gewünscht ist, ohne auf die hohe Kraftbelastung beim Einschlagen Rücksicht nehmen zu müssen. Außerdem hat der Block die vorteilhafte Wirkung, daß er ein Verdrehen der Zwischenwirbelendoprothese oder ein Aufklappen ihrer Elemente verhindert. Das erleichtert ein sicheres, einfaches und auch positionsgenaues Einsetzen der Zwischenwirbelendoprothese.

Der Block kann in Längsrichtung mittels einer Betätigungseinrichtung beweglich angeordnet sein. Er kann aber auch starr an dem Faßbereich angeordnet sein. Unter starr wird hierbei verstanden, daß der Block bei der Verwendung des Einsetzinstruments zum Halten und Einsetzen nicht bewegt wird; es soll aber nicht ausgeschlossen sein, daß er zu Einstellzwecken versetzt werden kann, z. B. zur Anpassung an eine andere Prothesengröße. Das kann beispielsweise durch eine Befestigung mittels einer Klemmschraube erreicht werden. Vorzugsweise ist der Block aber mittels einer Durchgangsschraube an dem Faßbereich befestigt.

Zweckmäßigerweise ist an dem Block eine Stange mit einer Handhabe im hinteren Bereich des Griffteils angeordnet. Sie vereinfacht das Aufbringen der Einsetzkraft auf das Einsetzinstrument und die Zwischenwirbelendoprothese. Vorzugsweise ist dazu die Handhabe als Schlagkopf ausgebildet.

Günstig ist es, den starren Block an dem Backeneinsatz anzuordnen. Eine Anpassung an unterschiedliche Zwischenwirbelendoprothesen kann damit auf einfache Weise durch Austausch des Backeneinsatzes erfolgen.

Die Erfindung wird nachfolgend unter Bezugnahme auf die Zeichnung erläutert, in der ein vorteilhaftes Ausführungsbeispiel dargestellt ist. Es zeigen:
- Fig. 1: eine Gesamtansicht des erfindungsgemäßen Anordnung von ihrer Oberseite;
- Fig. 2: eine Gesamtansicht des Einsetzinstruments von der Unterseite;
- Fig. 3: eine vergrößerte Teilansicht eines Backenteils des Einsetzinstruments in einem Längsachsenschnitt;
- Fig. 4: eine Teilansicht eines anderen Backeneinsatz;
- Fig. 5: eine Teilansicht des Einsetzinstruments mit daran angeordneter Zwischenwirbelendoprothese;
- Fig. 6: eine vergrößerte Teilansicht eines Backenteils einer anderen Ausführungsform des Einsetzinstruments;
- Fig. 7: eine vergrößerte Teilansicht eines Backenteils einer weiteren anderen Ausführungsform des Einsatzinstruments; und
- Fig. 8: eine Teilansicht eines Griffteils des Einsatzinstruments gemäß Fig. 7.

Bei dem dargestellten Ausführungsbeispiel handelt es sich um ein als Zange ausgeführtes Einsetzinstrument (in seiner Gesamtheit mit dem Bezugszeichen 1 versehen) und einer Zervikalprothesen 9 als Zwischenwirbelendoprothese. Sie ist zur Implantation in den Zwischenraum zweier benachbarter Wirbelkörper der Halswirbelsäule (nicht dargestellt) vorgesehen.

Die Zervikalprothese 9 besteht aus einer oberen Abschlußplatte 91 und einer unteren Abschlußplatte 92 mit einem dazwischen angeordneten Gleitkern 93. Die Zervikalprothese 9 ist zur Implantation in dem Zwischenraum zweier benachbarter wirbel der Halswirbelsäule eines Menschen vorgesehen. Dabei wird die obere Abschlußplatte 91 an der Unterseite des kranialen Wirbels und die untere Abschlußplatte 92 an der Oberseite des kaudalen Wirbels befestigt. Damit die Zervikalprothese 9 zum Einsetzen sicher von der Zange 1 aufgenommen werden kann, sind die obere und die untere Abschlußplatte 91, 92 mit Aufnahmeöffnungen versehen. Sie befinden im vorderen Bereich der Abschlußplatten im Bereich eines ventralen Befestigungsflansch 94, 95. Die der oberen Abschlußplatte 91 zugeordnete Aufnahmeöffnung ist als Rundbohrung 97 mit einer Ansenkung ausgeführt. Die der unteren Abschlußplatte 92 zugeordnete Aufnahmeöffnung ist als ein Schlitz 96 an der Seitenflanke der unteren Abschlußplatte 92 selbst und als ein Schlitz 96' augeführt, der an der Seitenflanke des Gleitkerns 93 ausgebildet ist. Die Schlitze 96 und 96' fluchten, so daß sie eine durchgehende Rinne ergeben. Es ist nicht zwingend erforderlich, daß der Schlitz 96, 96' sowohl in der unteren Abschlußplatte 92 wie auch dem Gleitkern 93 ausgebildet ist; es kann auch ausreichen, ihn nur in der Abschlußplatte 92 vorzusehen.

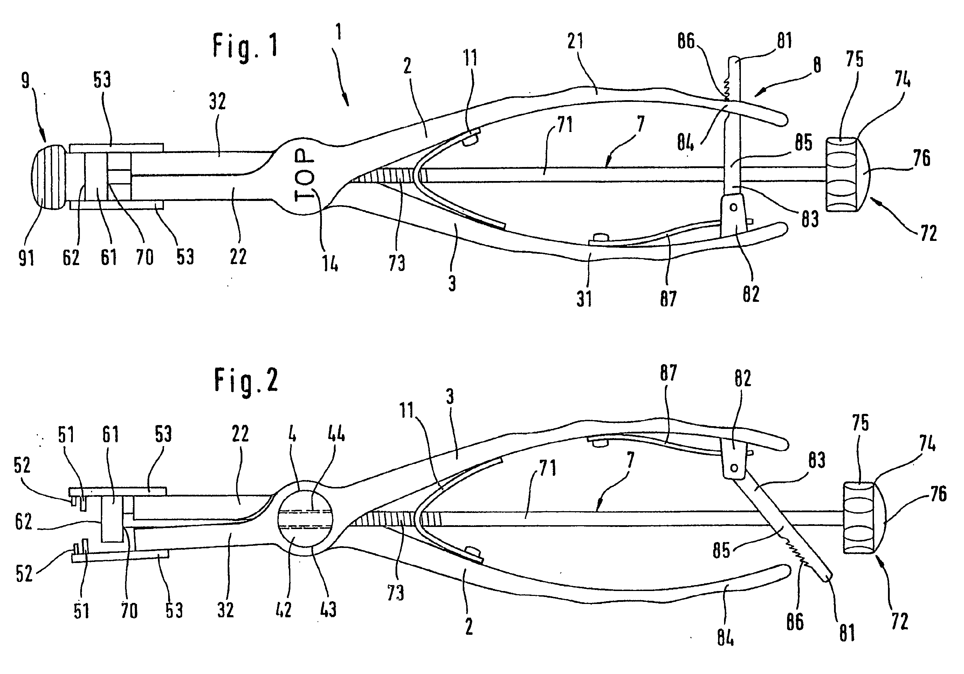

Die Zange 1 ist aus zwei Zangenhälften 2, 3 gebildet, die über ein Schwenkgelenk 4 beweglich miteinander verbunden sind. Die Zangenhälften 2, 3 weisen in ihrem hinteren Bereich jeweils ein Griffteil 21, 31 und in ihrem vorderen Bereich jeweils ein Backenteil 22, 32 auf. In dem Übergang zwischen den Griffteilen 21, 31 und den Backenteilen 22, 32 ist das Schwenkgelenk 4 angeordnet. Es ist gebildet durch einen Zapfen 42 an der Zangenhälfte 2 (in Fig. 1 erstreckt er sich aus der Zeichenebene nach oben heraus), der in einer passenden Öffnung 43 in dem mittleren Bereich der anderen Zangenhälfte 3 gelagert ist. Der Lagerzapfen 42 weist eine Durchgangsbohrung 44 auf, die von dem griffseitigen Bereich der Zangenhälften 2, 3 zu dem backenseitigen Bereich läuft. Sie wird später noch näher erläutert werden. Das Schwenklager 4 erlaubt es, die Griffteile 21, 31 der Zangenhälften 2, 3 aufeinander zu zu bewegen, damit sich die Backenteile 22, 32 schließen und umgekehrt.

Die Backenteile 22, 32 fungieren als Greifglieder. Sie weisen im vorderen Bereich an ihren einander zugewandten Innenseiten jeweils zwei in Spannrichtung 12 weisende Vorsprünge 51, 52 auf. Diese Vorsprünge sind nicht unmittelbar an den Backenteilen 22, 32, sondern an Backeneinsätzen 53 angeordnet, die mittels einer Schraube (nicht dargestellt) auswechselbar an den Außenseiten der Backenteile 22, 32 in einer entsprechenden Aufnahme befestigt sind. Jeder Backeneinsatz 53 weist je einen Vorsprung 51 und einen Vorsprung 52 auf. Der Vorsprung 51 ist stiftartig geformt und befindet sich im oberen Bereich, während der Vorsprung 52 plättchenartig geformt ist und sich im unteren Bereich des Backeneinsatzes 53 befindet. Die Abmessungen und die Anordnung der Vorsprünge 51, 52 sind auf entsprechende Aufnahmeöffnungen an den aufzunehmenden Zervikalprothesen 9 angepaßt. Das wird nachfolgend näher erläutert.

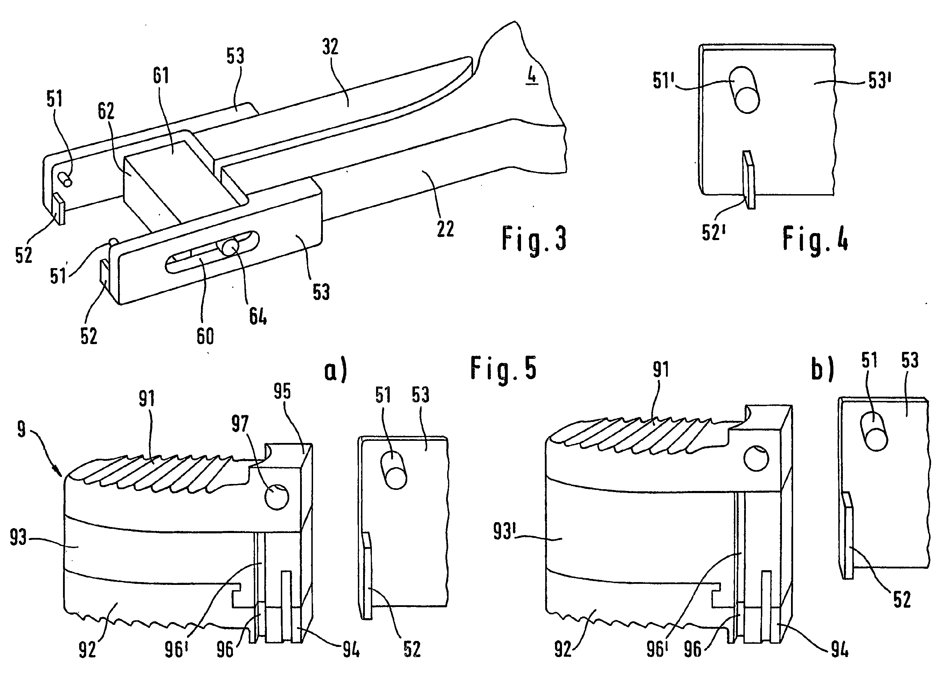

Die Rundbohrung 97 ist passend zu einem stiftartigen Vorsprung 51 der Zange 1 bemessen. Dank der Ansenkung kann der stiftartige Vorsprung 51 leicht in die Rundbohrung 97 eingeführt werden. Der Schlitz 96, 96' weist eine langgestreckte Form in Richtung zu oberen Abschlußplatte 91 auf. Der an der Zange 1 angeordnete plättchenartige Vorsprung 52 ist in seiner Breite auf die Weite des Schlitzes 96, 96' abgestimmt, so daß er entlang dem Schlitz geführt ist. Dadurch wird die relative Position der Zervikalprothese 9 in Bezug auf die Zange 1 durch den in die Rundbohrung 97 eingreifenden stiftartigen Vorsprung 51 festgelegt, während der plättchenartige Vorsprung 52 je nach der Dicke des Gleitkerns 93 an unterschiedlichen Stellen in den Schlitz 96, 96' einfaßt und somit einen Ausgleich für unterschiedliche Gleitkerne 93 ermöglicht. In Fig. 5a ist eine Zervikalprothese mittlerer Dicke und der vordere Bereich der Zange 1 mit den Vorsprüngen 51 und 52 dargestellt. Beim Aufnehmen der Zervikalprothese 9 durch die Zange 1 greift der stiftartige Vorsprung 51 in die Rundbohrung 97 und fixiert die Zervikalprothese gegenüber Verschiebungen. Der plättchenartige Vorsprung 52 greift in den Schlitz 96 der unteren Abschlußplatte 92 und in den sich unmittelbar anschließenden unteren Bereich des Schlitz 96' des Gleitkerns 93. Zum Vergleich ist in Fig. 5b eine Zervikalprothese 9' mit einem dickeren Gleitkern 93' dargestellt. Wieder greift der stiftartige Vorsprung 51 in die Aufnahmebohrung 97. Der plättchenartige Vorsprung 52 greift aber wegen des dickeren Gleitkerns 93' nicht mehr in den Schlitz 96 der unteren Abschlußplatte 92, sondern nur in den Schlitz 96' des Gleitkerns 93'. Damit wird die Zervikalprothese 9' trotz ihrer abweichenden Höhe ebenfalls sicher in einer definierten Position an der Zange 1 gehalten.

An dem Backenteil 22 ist eine Führungsschiene 60 angeordnet, die einen Block 61 längsverschieblich an der Zangenhälfte 2 in Vor- und Rückwärtsrichtung beweglich hält. Die Führungsschiene 60 ist als ein Langloch in dem Backeneinsatz 53 des Backenteils 22 ausgebildet. Eine seitlich in dem Block 61 angeordnete Madenschraube greift in das die Führungsschiene 60 bildende Langloch ein und führt den Block 61 in Längsrichtung. Anstelle des Langlochs können auch andere Führungselemente vorgesehen sein, die eine in Längsrichtung vor- und zurückbewegliche Führung des Blocks 61 erlauben, beispielsweise eine Schwalbenschwanzführung. Der Block 61 ist an seinem vorderen Ende mit einer Anschlagfläche 62 versehen, die zum Zusammenwirken mit der Zervikalprothese 9 ausgebildet ist.

An dem Block 61 greift eine Betätigungseinrichtung 7 an, die sich von dem hinteren Bereich des Blocks 61 durch die Durchgangsbohrung 44 in den Bereich zwischen den Griffteilen 21, 31 erstreckt. Die Betätigungseinrichtung 7 umfaßt ein Kopplungselement 70 mit dem Block 61, das in dem dargestellten Ausführungsbeispiel ein zur Übertragung von Schubkräften geeignetes Wirbellager ist, ferner eine Stange 71 und eine Handhabe 72 zur Betätigung. Im vorderen Bereich der Stange 71 ist ein Außengewinde 73 vorgesehen, das mit einem komplementären Innengewinde (nicht dargestellt) in der Durchgangsbohrung 44 des Zapfens 42 als instrumentenfeste Führung zusammenwirkt. Damit ist es ermöglicht, durch Drehen der Handhabe 72 die Stange 71 und damit über das Kopplungselement 70 den Block 61 entlang der Führungsschiene 60 vor- und zurückzubewegen. Die Handhabe 72 ist als Drehknopf ausgebildet, der an seiner äußeren Mantelfläche 74 zur Bereitstellung einer guten Greifmöglichkeit für den Operateur eine geeignete Oberflächengestaltung, wie eine Grobriffelung 75, aufweist.

Das hintere Ende der Handhabe 72 ist mit einer konvexen Wölbung 76 versehen. Sie dient als Schlagkopf für die Betätigungseinrichtung 7. Sie überträgt Impulse von auf die Wölbung 76 des Schlagkopfes einwirkenden Schlägen über die Stange 71 der Betätigungseinrichtung 7, das schubfeste Wirbellager 70 und den Block 61 auf dessen Anschlagfläche 62.

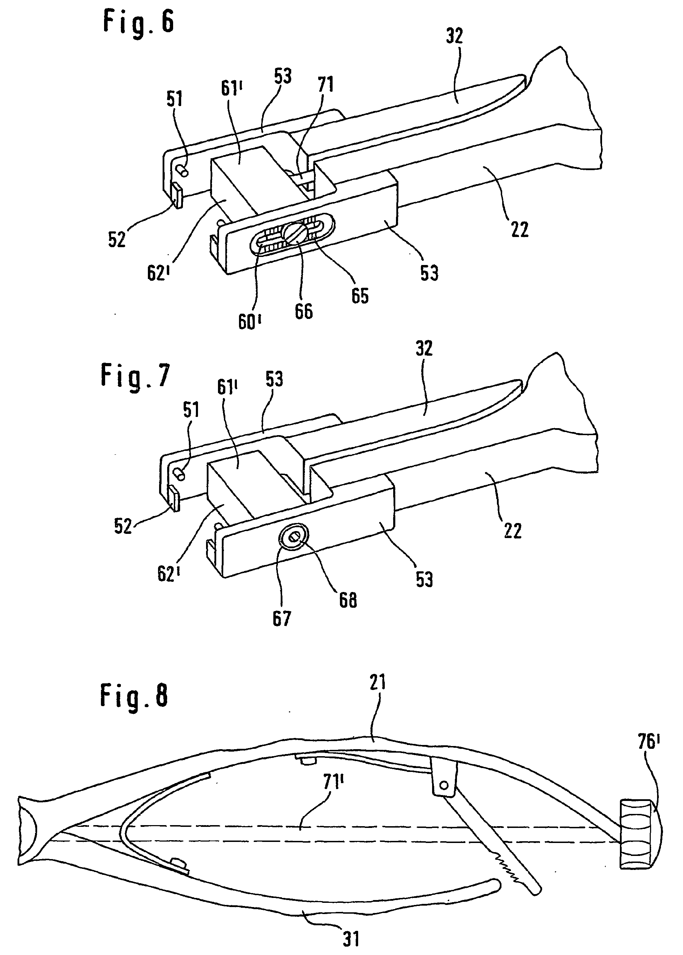

Alternativ kann auch vorgesehen sein, statt des längsbeweglichen Blocks 61 einen starr an der Zangenhälfte 2 angeordneten Block 61' vorzusehen und so anzuordnen, daß seine Anschlagläche 62' an der gehaltenen Zervikalprothese 9 anliegt. Dies ist insbesondere dann von Interesse, wenn die verwendeten Zervikalprothesen 9 sich zwar in ihren Höhen- und/oder Breitenabmessungen unterscheiden können, aber identische Längsabmessungen aufweisen. Da die Zervikalprothese 9 durch die erfindungsgemäßen Gestaltung der Aufnahmeöffnungen 96, 97 und Haltevorsprünge 51, 52 in einer definierten Position an der Zange 1 gehalten ist, ist eine Längsbeweglichkeit des Blocks nicht zwingend erforderlich. Dank der definierten Position ist es auch mit starrem Block 61' möglich, daß er mit seiner Anschlagfläche 62' an der Zervikalprothese 9 anliegt. Die Einsetzkräfte, insbesondere die Schlagkräfte, können so sicher und schonend wie bei der Ausführungsform mit beweglichem Block übertragen werden. Es versteht sich, daß bei dem starr angeordneten Block 61' eine Führung 60 entbehrlich ist. Sie kann aber auch beibehalten sein, wobei dann die Befestigung des Blocks 61' mittels einer Klemmschraube 66 erfolgen kann (Fig. 6). Zur besseren Befestigen ist das Langloch 60 mit einer Riffelung 65 für die Klemmschraube 66 versehen. Einfacher ist es, den starren Block 61' an dem Faßteil mittels einer Verschweißung oder Verschraubung an dem Faßteil 22 bzw. dessen Backeneinsatz 53 zu befestigen. Im letzteren Fall ist vorzugsweise eine versenkt in einer Bohrung 67 des Backeneinsatzes 53 angeordnete Schraube 68 vorgesehen (siehe Fig. 7). Da die Betätigungseinrichtung 7 keine Längsverstellung mehr zu bewirken braucht, können das Außengewinde 73 an der Stange 71 sowie das Gegengewinde in der Durchgangsbohrung 44 entfallen; die Durchgangsbohrung 44 fungiert nur noch als Führung für die Stange 71. Das schubfeste Wirbellager 70 braucht auch keine Drehbewegung zu übertragen und kann durch eine starre schubfeste Verbindung ersetzt werden, bspw. eine Verschraubung oder Verschweißung. Die Handhabe 72 fungiert weiter als Schlagkopf und weist dazu vorzugsweise die Wölbung 76 auf. Diese Ausführungsform ist durch den Verzicht auf die längsbewegliche Anordnung des Blocks 61' und den Entfall der Betätigungseinrichtung zum Bewegen des Blocks und weniger aufwendig in der Herstellung und einfacher in der Bedienung.

Bei einer anderen Ausführungsform ist zur weiteren Vereinfachung außerdem vorgesehen, das hintere Ende der Griffteils 21 als Schlagkopf auszubilden und mit einer Wölbung 76' zu versehen. Gegebenenfalls kann eine Verstärkungsstange 71' vorgesehen sein, die das hintere Ende des Griffteils 21 mit seinem vorderen Ende verbindet.

Bei den Ausführungsformen mit starrem Block 61' werden die Impulse von der Wölbung 76, 76' über die Stange 71, 71' und die Zangenhälfte 2 auf den Block 61' und dessen Anschlagfläche 62' übertragen.

Im hinteren Bereich der Zange 1 ist eine Rasteinrichtung 8 für die Griffteile 21, 31 vorgesehen. Sie umfaßt ein schwenkbewegliches Verrastungselement 83 und eine Rastklinke 84 (die jeweils gegenüberliegend an den Griffteilen 21, 31 angeordnet sind), eine Löseeinrichtung 81, einen Fuß 82 und eine Feder 87. Das hintere Ende des Griffteils 21 ist als Gabel ausgeführt, wobei die Rastklinke 84 durch eine Anschrägung des Fußpunkt der Gabel gebildet ist. Das Verrastungselement 83 ist durch den Fuß 82 in der durch die Griffteile 21, 31 aufgespannten Ebene gelagert. Die Feder 87 ist als Blattfeder ausgebildet und wirkt auf das in dem Fuß 82 gelagerte Ende des Verrastungselements 83 so ein, daß es nach vorne zur Rastklinke 84 gedrückt wird. Ausgehend von dem Fuß 82 weist das Verrastungselement 83 einen breiten Bereich und einen schmalen Bereich auf. In seinem schmalen Bereich weist das Verrastungselement 83 an seiner Vorderseite eine Verzahnung 86 auf, in welche im geschlossenen Zustand der Zange 1 die Rastklinke 84 greift und verrastet, so daß die Griffteile 21, 31 sich nicht voneinander wegbewegen können und damit das Einsetzinstrument 1 gegenüber einem versehentlichen Aufspringen gesichert ist. Dadurch können auch erhebliche Belastungen, wie bspw. Hammerschläge auf die Wölbung 76, auf der Zange 1 ausgeübt werden, ohne daß ein versehentliches Öffnen zu befürchten ist und ohne daß der Operateur die Griffteile 21, 31 mit Handkraft gegenüber einem unerwünschten Öffnen zu sichern braucht. Zum Öffnen der Zange 1 nach erfolgter Implantation wird durch Druck nach hinten auf das Auslöseelement 81 das Verrastungselement 83 nach hinten geschwenkt, wodurch die Rastklinke 84 von dem Verrastungselement 83 freikommt und somit die Griffteile 21, 31 sich unter der Wirkung der Feder 11 auseinander bewegen. Im geöffneten Zustand der Zange 1 ist das Verrastungselement 83 gegen die Kraft der Feder 87 nach hinten geschwenkt. In dem breiten Bereich des Verrastungselements 83 ist eine als Langloch ausgebildete Führung 85 vorgesehen, die dazu dient, die Stange 71 auch bei geöffneter Zange 1 in einer definierten Position in der Längsachse 10 zu halten und ein Ausknicken der Stange 71 auch unter hoher Schlagbelastung zu vermeiden.

An dem Griffteil 31 ist ferner eine Blattfeder 11 befestigt, die um die Stange 71 herum zu dem anderen Griffteil 21 geführt ist. Bei geschlossener Zange 1 steht diese Blattfeder 11 unter Spannung und bewirkt, daß das Einsetzinstrument 1 nach dem Lösen des Verrastungselements 83 selbsttätig öffnet, um ein Abnehmen zu ermöglichen.

Nachfolgend soll das Zusammenwirken mit der Zervikalprothese 9 beschrieben werden. Zum Aufnehmen der Zervikalprothese 9 mit der Zange 1 wird die Zervikalprothese 9 in dem Bereich zwischen die Backenteile 22, 32 gebracht und die Zange 1 geschlossen, wodurch sich die Backenteile 22, 32 aufeinander zu bewegen. Dabei greifen die Vorsprünge 51, 52 in die entsprechenden Aufnahmeöffnungen der beiden Abschlußplatten 91, 92 ein, wobei die Stifte 51 in die Bohrung 97 und die Plättchen 52 in die Schlitze 96, 96' eingreifen. Dadurch wird die Zervikalprothese 9 in Spannrichtung spielfrei an der Zange 1 gehalten. Die unterschiedliche Ausführung der Vorsprünge 51, 52 und der Aufnahmeöffnungen in Gestalt der Bohrungen 97 und Schlitze 96 stellt sicher, daß die Zervikalprothese 9 nur mit der richtigen Orientierung an der Zange 1 gehalten werden kann. Ist die Zange 1 wie in dem dargestellten Ausführungsbeispiel auch zusätzlich mit einer Markierung 14 für die Oberseite versehen, so sind Fehlimplantationen aufgrund verkehrter Orientierung der Zervikalprothese 9 praktisch ausgeschlossen. Nachdem auf diese Weise die Zervikalprothese 9 an der Zange 1 in der richtigen Orientierung aufgenommen wurde, kann mittels der Betätigungseinrichtung 7 durch Drehen der Handhabe 72 die Stange 71 nach vorne bewegt werden, so daß der Block 61 mit seiner Anschlagsfläche 62 von hinten an den Flansch 94, 95 der Zervikalprothese 9 zur Anlage kommt. Dabei spannt der Block 61 die Zervikalprothese 9 gegen die Vorsprünge 51, 52 und richtet so die Zervikalprothese 9 in eine definierte Position aus. Eventuell vorhandenes Spiel in Längsachsenrichtung zwischen den Vorsprüngen 51, 52 und den Bohrungen 97 und Schlitzen 96 wird auf diese Weise herausgedrückt. Die Zervikalprothese 9 ist damit sicher und positionsgenau an dem Einsetzinstrument 1 gehalten. Außerdem bewirkt das Anliegen des Blocks 61 an den Flanschen 93, 94 der beiden Abschlußplatten 91, 92, daß sich die beiden Abschlußplatten 91, 92 an ihrem vorderen Ende voneinander wegbewegen. Dadurch wird ein Aufklappen der Zervikalprothese 9, das ein erfolgreiches Einbringen in den Zwischenwirbelraum verhindern würde, ausgeschlossen.

Weiter ist es ermöglicht, Zervikalprothesen mit anderer Höhe ohne Veränderungen an der Zange 1 zu implantieren. In Fig. 5 sind Zervikalprothese 9, 9' dargestellt, die von denen eine einen dickeren Gleitkern 93' aufweist. Er ist (ebenso wie der Gleitkern 93) mit einem Schlitz 96' versehen, der mit dem Schlitz 96 der unteren Abschlußplatte 92 fluchtet. Diese Gestaltung der Aufnahmeöffnung an der unteren Abschlußplatte 92 als Schlitz 96 und seine Fortsetzung als Schlitz 96' in dem Gleitkern 93' ermöglichen es, die dickere Zervikalprothese 9' mit derselben Zange 1 mit unveränderter Anordnung der Vorsprünge 51, 52 zu greifen und sicher zu halten. Die Positionierungsgenauigkeit ist dabei durch die stiftartigen Vorsprünge 51, die in die Bohrungen 97 eingreifen, gesichert.

Bei Bedarf können aber auch andere Backeneinsätze 53' vorgesehen sein, die eine andere Anordnung der Vorsprünge 51', 52' aufweisen, wie sie in Fig. 4 dargestellt sind. In dem dargestellten Beispiel stehen die Vorsprünge näher zusammen und sind in einer Ebene. Damit kann die Zange 1 an andere Zwischenwirbelendoprothesen angepaßt werden, z. B. an besonders kleine zur Therapie von Kindern.

Der Block 61 stellt mit seiner Anschlagfläche 62 eine hinreichend groß dimensionierte Kraftübertragungsfläche zur Verfügung, um die auf die Wölbung 76 als Schlagkopf aufgebrachten Impulse auf die Zervikalprothese 9 zu übertragen. Das hat den großen Vorteil, daß die im Interesse einer genauen Positionierung feingliedrig ausgeführten Vorsprünge 51, 52 nicht die Schlagkräfte übertragen müssen, so daß die Gefahr eines Verbiegens oder gar Abbrechens der Vorsprünge 51, 52 aufgrund Überbelastung auch bei hoher Schlagbelastung dank des die Kraftübertragung übernehmenden Blocks 61 bzw. 61' mit seiner Anschlagfläche 62 ausgeschlossen ist.

Die erfindungsgemäße Zange 1 ermöglicht eine verwechslungssichere und positionsgenaue Anordnung der Zervikalprothese 9 an der Zange 1, wobei ein unerwünschtes Aufklappen der Zervikalprothese 9 verhindert wird. Ferner ermöglicht sie dank des Blocks 61 bzw. 61' mit der Anschlagfläche 62 auch bei kleindimensionierten Zangen 1 die Übertragung hoher Kräfte. Damit ist ein sicheres Implantieren der Prothese gewährleistet. Die kleine Dimensionierung.hat ferner den Vorteil, daß sie dem Operateur gute Sicht- und Zugangsbedingungen auf den Implantationsort gibt.

## Patentansprüche

1. Anordnung aus einer mehrteiligen Zwischenwirbelendoprothese (9), die eine obere und untere Abschlußplatte (91, 92) und einen Gleitkern (93) dazwischen aufweist, wobei jeder Abschlußplatte (91, 92) ein Paar Aufnahmeöffnungen (96, 97) oder - vorsprünge zugeordnet ist, und einem Einsetzinstrument (1), das einen Griffbereich (21, 31) und einen Faßbereich (22, 32) mit Haltevorsprüngen (51, 52) oder -öffnungen aufweist, die zum Halten der Zwischenwirbelendoprothese (9) an dem Einsetzinstrument (1) in die Aufnahmeöffnungen (96, 97) oder -vorsprünge einbringbar sind,
**dadurch gekennzeichnet, daß**
die Aufnahmeöffnungen (96, 97) oder -vorsprünge in lateralen Seitenflächen der Zwischenwirbelendoprothese (9) angeordnet sind und zumindest das einer der Abschlußplatten (92) zugeordnete Paar von AufnahmeÖffnungen (96) oder -vorsprünge eine in Richtung zur anderen Abschlußplatte (91) ausgedehnte Form aufweist.

2. Anordnung nach Anspruch 1,
**dadurch gekennzeichnet, daß**
die eine ausgedehnte Form aufweisende Aufnahmeöffnung (96) ein Schlitz ist.

3. Anordnung nach Anspruch 1 oder 2,
**dadurch gekennzeichnet, daß**
die eine ausgedehnte Form aufweisende Aufnahmeöffnung (96) sich über die ganze Höhe der zugeordneten Abschlußplatte (92) erstreckt.

4. Anordnung nach einem der vorhergehenden Ansprüche,
**dadurch gekennzeichnet, daß**
die eine ausgedehnte Form aufweisende Aufnahmeöffnung (96') sich über einen Teil der Höhe des Gleitkerns (93) erstreckt.

5. Anordnung nach einem der vorhergehenden Ansprüche,
**dadurch gekennzeichnet, daß**
daß die eine ausgedehnte Form aufweisende Aufnahmeöffnung (96') sich über die ganze Höhe des Gleitkerns (93) erstreckt.

6. Anordnung nach einem der vorhergehenden Ansprüche,
**dadurch gekennzeichnet, daß**
die eine ausgedehnte Form aufweisende Aufnahmeöffnung (96) sich mit zunehmender Tiefe verjüngt.

7. Anordnung nach einem der vorhergehenden Ansprüche;
**dadurch gekennzeichnet, daß**
die der anderen Abschlußplatte (91) zugeordnete Aufnahmeöffnung (97) eine konzentrierte Form aufweist.

8. Anordnung nach Anspruch 7,
**dadurch gekennzeichnet, daß**
sie als Rundbohrung ausgeführt ist.

9. Anordnung nach einem der vorhergehenden Ansprüche,
**dadurch gekennzeichnet, daß**
die Haltevorsprünge plättchenartig (52) und stiftartig (51) ausgeführt sind.

10. Anordnung nach einem der vorhergehenden Ansprüche,
**dadurch gekennzeichnet, daß**
die Halteöffnungen an dem Einsetzinstrument (1) und die Aufnahmevorsprünge an der Zwischenwirbelendoprothese (9) angeordnet sind.

11. Anordnung nach einem der vorhergehenden Ansprüche,
**dadurch gekennzeichnet, daß**
für die Zwischenwirbelendoprothese (9, 9') verschiedene Größen mit unterschiedlich dicken Gleitkern (93, 93') vorgesehen sind.

12. Anordnung nach einem der vorhergehenden Ansprüche,
**dadurch gekennzeichnet, daß**
an einem Faßteil ein Block (61') mit einer Anschlagfläche (62) zur Anlage an die Zwischenwirbelendoprothese (9) vorgesehen ist, der mit einem Kraftaufnahmeteil zur Ausübung einer Einsetzkraft auf die Zwischenwirbelendoprothese (9) verbunden ist.

13. Anordnung nach Anspruch 12,
**dadurch gekennzeichnet, daß**
der Block (61') starr an dem Faßteil (22) angeordnet ist.

14. Anordnung nach Anspruch 13,
**dadurch gekennzeichnet, daß**
der Block (61') mittels einer Durchgangsschraube (68) befestigt ist.

15. Anordnung nach Anspruch 13,
**dadurch gekennzeichnet, daß**
der Block (61') mittels einer Klemmschraube (66) befestigt ist.

16. Anordnung nach einem der Ansprüche 12 bis 15,
**dadurch gekennzeichnet, daß**
an dem Block (61') eine Stange (71) mit einer Handhabe (72) im hinteren Bereich des Griffteils (21) angeordnet ist.

17. Anordnung nach Anspruch 16,
**dadurch gekennzeichnet, daß**
die Handhabe als Schlagkopf (76) ausgebildet ist.

18. Anordnung nach einem der Ansprüche 13 bis 17
**dadurch gekennzeichnet, daß**
der Block (61') an einem Backeneinsatz (53) angeordnet ist.

## Claims

1. Arrangement comprising a multipart intervertebral endoprosthesis (9), having an upper and lower end plate (91, 92) and an intermediate sliding core (93), in which each end plate (91, 92) is assigned a pair of receiving apertures (96, 97) or protrusions, and an insertion instrument (1), which has a handle region (21, 31) and a gripping region (22, 32) with retaining protrusions (51, 52) or apertures, which can be inserted into the receiving apertures (96, 97) or protrusions for holding the intervertebral endoprosthesis (9) on the insertion instrument (1), **characterized in that** the receiving apertures (96, 97) or protusions are arranged in lateral side faces of the intervertebral endoprosthesis (9) and at least the pair of receiving apertures (96) or protusions assigned to one of the end plates (92) has a form expanding in the direction of the other end plate (91).

2. Arrangement according to Claim 1, **characterized in that** the receiving aperture (96) having an expanded form is a slot.

3. Arrangement according to Claim 1 or 2, **characterized in that** the receiving aperture (96) having an expanded form extends over the entire height of the assigned end plate (92).

4. Arrangement according to any one of the preceding claims, **characterized in that** the receiving aperture (96') having an expanded form extends over part of the height of the sliding core (93).

5. Arrangement according to any one of the preceding claims, **characterized in that** the receiving aperture (96') having an expanded form extends over the entire height of the sliding core (93).

6. Arrangement according to any one of the preceding claims, **characterized in that** the receiving aperture (96) having an expanded form tapers with increasing depth.

7. Arrangement according to any one of the preceding claims, **characterized in that** the receiving aperture (97) assigned to the other end plate (91) has a concentrated form.

8. Arrangement according to Claim 7, **characterized in that** it is designed as a round bore.

9. Arrangement according to any one of the preceding claims, **characterized in that** the retaining protrusions are designed platelike (52) and pinlike (51).

10. Arrangement according to any one of the preceding claims, **characterized in that** the receiving apertures are arranged on the insertion instrument (1) and the retaining protrusions are arranged on the intervertebral endoprosthesis (9).

11. Arrangement according to any one of the preceding claims, **characterized in that** different sizes with a sliding core (93, 93') of varying thickness are provided for the intervertebral endoprosthesis (9, 9').

12. Arrangement according to any one of the preceding claims, **characterized in that** a block (61') with a stop face (62) for bearing on the intervertebral endoprosthesis (9), connected to a force transducer part for exerting an insertion force on the intervertebral endoprosthesis (9), is provided on a gripping region.

13. Arrangement according to Claim 12, **characterized in that** the block (61') is arranged rigidly on the gripping region (22).

14. Arrangement according to Claim 13, **characterized in that** the block (61') is affixed by means of a through bolt (68).

15. Arrangement according to Claim 13, **characterized in that** the block (61') is affixed by means of a clamping screw (66).

16. Arrangement according to any one of Claims 12 to 15, **characterized in that** a rod (71) with a handle (72) in the rear region of the handle part (21) is arranged on the block (61').

17. Arrangement according to Claim 16, **characterized in that** the handle is designed as impact head (76).

18. Arrangement according to any one of Claims 13 to 17, **characterized in that** the block (61') is arranged on a cheek insert (53).

## Revendications

1. Agencement constitué d'une endoprothèse intervertébrale (9) en plusieurs parties, qui présente une plaque de terminaison supérieure et une plaque de terminaison inférieure (91, 92) et un noyau glissant (93) entre elles, à chaque plaque de terminaison (91, 92) étant associée une paire d'ouvertures ou de saillies de réception (96, 97), et d'un instrument d'insertion (1) qui présente une zone de préhension (21, 31) et une zone de saisie (22, 32) avec des saillies ou des ouvertures de retenue (51, 52) qui peuvent être insérées (1) dans les ouvertures ou les saillies de réception (96, 97) pour retenir l'endoprothèse intervertébrale (9) sur l'instrument d'insertion,
**caractérisé en ce que**
les ouvertures ou saillies de réception (96, 97) sont disposées dans des surfaces latérales de l'endoprothèse intervertébrale (9) et au moins la paire d'ouvertures ou de saillies (96) associée à l'une des plaques de terminaison (92) présente une forme étirée dans la direction de l'autre plaque de terminaison (91).

2. Agencement selon la revendication 1,
**caractérisé en ce que**
l'ouverture de réception (96) présentant une forme étirée est une fente.

3. Agencement selon la revendication 1 ou 2,
**caractérisé en ce que**
l'ouverture de réception (96) présentant une forme étirée s'étend sur toute la hauteur de la plaque de terminaison associée (92).

4. Agencement selon l'une quelconque des revendications précédentes,
**caractérisé en ce que**
l'ouverture de réception (96') présentant une forme étirée s'étend sur une partie de la hauteur du noyau glissant (93).

5. Agencement selon l'une quelconque des revendications précédentes,
**caractérisé en ce que**
l'ouverture de réception (96') présentant une forme étirée s'étend sur toute la hauteur du noyau glissant (93).

6. Agencement selon l'une quelconque des revendications précédentes,
**caractérisé en ce que**
l'ouverture de réception (96) présentant une forme étirée se rétrécit avec une profondeur croissante.

7. Agencement selon l'une quelconque des revendications précédentes,
**caractérisé en ce que**
l'ouverture de réception (97) associée à l'autre plaque de terminaison (91) présente une forme concentrée.

8. Agencement selon la revendication 7,
**caractérisé en ce**
**qu'**il est réalisé sous forme d'alésage rond.

9. Agencement selon l'une quelconque des revendications précédentes,
**caractérisé en ce que**
les saillies de retenue sont réalisées sous forme de petites plaques (52) et sous forme de broches (51).

10. Agencement selon l'une quelconque des revendications précédentes,
**caractérisé en ce que**
les ouvertures de retenue sont disposées sur l'instrument d'insertion (1) et les saillies de réception sont disposées sur l'endoprothèse intervertébrale (9).

11. Agencement selon l'une quelconque des revendications précédentes,
**caractérisé en ce que**
pour l'endoprothèse intervertébrale (9, 9'), on prévoit différentes tailles avec des noyaux glissants (93, 93') de différentes épaisseurs.

12. Agencement selon l'une quelconque des revendications précédentes,
**caractérisé en ce que**
l'on prévoit sur une partie de saisie un bloc (61') avec une surface de butée (62) pour l'application contre l'endoprothèse intervertébrale (9), qui est connecté à une partie de réception de force pour exercer une force d'insertion sur l'endoprothèse intervertébrale (9).

13. Agencement selon la revendication 12,
**caractérisé en ce que**
le bloc (61') est disposé rigidement sur la partie de saisie (22).

14. Agencement selon la revendication 13,
**caractérisé en ce que**
le bloc (61') est fixé au moyen d'une vis traversante (68).

15. Agencement selon la revendication 13,
**caractérisé en ce que**
le bloc (61') est fixé au moyen d'une vis de serrage (66).

16. Agencement selon l'une quelconque des revendications 12 à 15,
**caractérisé en ce que**
l'on dispose sur le bloc (61') une tige (71) avec une manette (72) dans la région arrière de la partie de préhension (21).

17. Agencement selon la revendication 16,
**caractérisé en ce que**
la manette est réalisée sous forme de tête de frappe (76).

18. Agencement selon l'une quelconque des revendications 13 à 17,
**caractérisé en ce que**
le bloc (61') est disposé sur un insert de mâchoire (53).
